Europäisches Patentamt

European Patent Office

Office européen des brevets

(11) Publication number: **0 310 034**
**A2**

# EUROPEAN PATENT APPLICATION

(21) Application number: 88115998.2

(51) Int. Cl.⁴: **C12P 13/04**

(22) Date of filing: 28.09.88

(30) Priority: 28.09.87 JP 244816/87

(43) Date of publication of application:
05.04.89 Bulletin 89/14

(84) Designated Contracting States:
**DE FR GB NL**

(71) Applicant: KURARAY CO., LTD.
**1621 Sakazu**
**Kurashiki-City Okayama Prefecture 710(JP)**

(72) Inventor: **Matsunaga, Tadashi**
**2-41-13, Saiwai-cho**
**Fuchu-City Tokyo(JP)**
Inventor: **Kitamura, Takanori**
**382-7, Futsukaichi**
**Kurashiki City Okayama(JP)**

(74) Representative: **Vossius & Partner**
**Siebertstrasse 4 P.O. Box 86 07 67**
**D-8000 München 86(DE)**

(54) Process for production of L-phenylalanine lower alkyl esters.

(57) A novel process for the production of an L-phenylalanine lower alkyl ester in a good yield and at a high production rate is provided which comprises permitting a microorganism which belongs to the genus Nocardia and is capable of producing an L-phenylalanine lower alkyl ester from the corresponding lower alkyl ester of phenylpyruvic acid and an amino group donor to act on a lower alkyl ester of phenylpyruvic acid and an amino group donor in the presence of hydrogen, water and an organic solvent.

## Process for production of L-phenylalanine lower alkyl esters

The present invention relates to a process for producing L-phenylalanine lower alkyl esters. More particularly, the invention relates to a process for producing an L-phenylalanine lower alkyl ester which comprises permitting a microorganism which belongs to the genus Nocardia and is capable of producing an L-phenylalanine lower alkyl ester from the corresponding lower alkyl ester of phenylpyruvic acid and an amino group donor to act upon a lower alkyl ester of phenylpyruvic acid and an amino group donor in the presence of hydrogen, water and an organic solvent.

Among the hithereto-known processes for the production of L-phenylalanine lower alkyl esters are the process which comprises refluxing L-phenylalanine in methanol in the presence of concentrated sulfuric acid and acetonitrile to produce L-phenylalanine methyl ester (JP-A-80013/1974) and the process which comprises refluxing L-phenylalanine in methanol in the presence of an equimolar amount of dimethyl sulfate to give methyl hydrogen sulfate salt of L-phenylalanine methyl ester and treating the salt with an aqueous solution of sodium hydroxide to provide L-phenylalanine methyl ester (EP-A-74,968 and JP-A-500442/1983).

Furthermore, the present inventors previously reported a process for producing L-phenylalanine which comprises permitting a microorganism which belongs to the genus Nocardia and is capable of producing L-phenylalanine from phenylpyruvic acid or a salt thereof and an amino group donor to act on an aqueous solution containing phenylpyruvic acid or a salt thereof and an ammonium salt or ammonia as amino group donor in a hydrogen gas atmosphere [cf. Abstracts of Papers, the 60th Annual Meeting of the Society of Fermentation Technology, Japan, 1985, p. 160; Abstracts of Papers, the 61th Annual Meeting of the Agricultural Chemical Society of Japan, 1986, p. 540; and EP-A-215,414].

In the above processes for producing L-phenylalanine lower alkyl esters from L-phenylalanine, the racemization and consequent loss of L-phenylalanine is inevitable during the course of esterification reaction. There fore, it is desirable that the production of L-phenylalanine lower alkyl esters be accomplished by a synthetic route not involving L-phenylalanine.

The present inventors have found that when a microorganism belonging to the genus Nocardia is permitted to act on an aqueous solution containing a lower alkyl ester of phenylpyruvic acid, in lieu of phenylpyruvic acid or a salt thereof, and an amino group donor in the above-mentioned process for producing L-phenylalanine, the rate of production of the desired L-phenylalanine lower alkyl ester is extremely low as will be apparent from the comparative example given hereinafter.

The primary object of the present invention is to provide a process for producing an L-phenylalanine lower alkyl ester at a high production rate and in a good yield, directly from a lower alkyl ester of phenylpyruvic acid and an amino group donor. This object as well as other objects and advantages of the present invention will become apparent to those skilled in the art from the following description.

The present invention provides a process for producing an L-phenylalanine lower alkyl ester which comprises permitting a microorganism which belongs to the genus Nocardia and is capable of producing an L-phenylalanine lower alkyl ester from the corresponding lower alkyl ester of phenylpyruvic acid and an amino group donor to act upon a lower alkyl ester of phenylpyruvic acid and an ammonium salt or ammonia as amino group donor in the presence of hydrogen, water and an organic solvent.

Representative strains of the above-mentioned microorganism belonging to the genus Nocardia and capable of producing an L-phenylalanine lower alkyl ester from the corresponding lower alkyl ester of phenylpyruvic acid and an amino group donor have been deposited at the Fermentation Research Institute, Agency of Industrial Science and Technology, Japan (hereinafter FERM). These strains are Nocardia opaca C-8-5 (FERM BP-1119), Nocardia coeliaca C-7-5 (FERM BP-1117) and Nocardia erythropolis C-6-2 (FERM BP-1118) and their bacteriological characteristics are as described in EP-A-215,414.

In the practice of the invention, the above-mentioned microorganisms belonging to the genus Nocardia and capable of producing an L-phenylalanine lower alkyl ester from the corresponding lower alkyl ester of phenylpyruvic acid and an amino group donor are cultivated in a nutrient medium, and the culture broth , living cells separated from said broth or dried cells derived therefrom are used. The cultivation of such microorganisms is carried out in the same manner as in the cultivation of microorganisms in general, generally in the manner of shake culture or submerged culture with aeration and agitation, using a liquid medium. As the nutrient medium, any medium containing nutrient sources utilizable by the above microorganisms can be used. Usable carbon sources are, for example, glucose, sucrose, maltose, peptone, meat extract, yeast extract, corn steep liquor and glycerol. These are used either alone or in combination, generally in a concentration of about 0.1-5% by weight. Usable nitrogen sources are such inorganic nitrogen sources as ammonium nitrate, ammonium sulfate and ammonium chloride and such organic nitrogen sources as peptone, meat extract, yeast extract and corn steep liquor. In addition, inorganic salts such as dipotassium hydrogen phosphate, potassium dihydrogen phosphate, magnesium sulfate and sodium chloride may be added. For enhancing the ability of the above-mentioned microorganisms to produce an L-phenylalanine lower alkyl ester, it is preferable to add the L, D or D/L forms of phenylalanine compounds such as methyl and other esters of phenylalanine, phenylalanine, phenylalanine amide and the like, and/or phenylpyruvic acid compounds such as methyl and other esters of phenylpyruvic acid, phenylpyruvic acid and the like to the medium in a concentration of about 0.1-1% by weight. Particularly, the use of esters of phenylalanine gives better results. The cultivation conditions are not critical. However, the cultivation is carried out in the manner of shake culture or submerged culture with aeration and agitation generally at 25-37°C, preferably at 28-35°C, and at a pH of 7-8 for about 4-24 hours. After cultivation, cells can be separated from the culture broth with ease by filtration or centrifugation, for instance. The living cells thus harvested may be dried by a conventional method to give dry cells.

The thus-obtained living or dried cells are preferably immobilized to a support prior to use. The immobilization of the living or dried cells can be performed by a conventional method of immobilizing microorganisms in general, for example by suspending the living or dried cells in water, isotonic sodium chloride solution or a buffer, such as tris buffer, citrate buffer or tricine buffer, admixing a polymerizable monomer or a polymerizable prepolymer with the suspension and allowing the polymerization reaction to proceed or by admixing a polymer with such suspension and coagulating the polymer with the cells. The immobilized living or dried cells can maintain their enzyme activity for a long period of time and can be easily recovered from the reaction mixture for reuse, as mentioned later herein. Examples of the support are polyacrylamide, carrageenin, fibroin, gelatin, collagen, agar, alginic acid salts, polyvinyl alcohol, diethylaminoethyl-Sephadex, and photocurable resins based on such prepolymers as diacrylates of polyethylene glycol or polypropylene glycol. These supports are used in any appropriate form and shape, for example in a granular, filmy or fibrous form. For efficient use of the immobilized cells, it is generally preferable that the support has a larger specific surface area. When the ease of forming and handling of the support, among others, is also taken into consideration, the support should preferably be granular, more preferably spherical. When spherical, the support should preferably have an average diameter of about 0.1-10 mm to give favorable results. The concentration of the living or dried cells in the support on a dry cell basis is generally about 1-100 grams, preferably about 5-50 grams, per liter of the support. For enhancing the ability of such immobilized cells to produce an L-phenylalanine lower alkyl ester, it is preferable to treat such immobilized cells with an aqueous solution containing at least one member selected from the group consisting of metal salts such as zinc chloride, magnesium chloride, etc.; phenylalanine compounds such as L-, D- and D/L-forms of methyl and other esters of phenylalanine, phenylalanine, phenylalanine amide, etc., and phenylpyruvic acid compounds such as methyl and other esters of phenylpyruvic acid, phenylpyruvic acid, etc. For example, such treatment is carried out by immersing the immobilized cells in a nutrient medium such as mentioned above or a buffer such as tris buffer, citrate buffer or tricine buffer, each containing at least one member selected from the group consisting of said metal salts, phenylalanine compounds and phenylpyruvic acid compounds, generally at about 25 to 40°C, preferably at 30-38°C, and within the pH range of about 7 to 8 for a time period ranging from about 2 to 50 hours. The metal salts are incorporated at a final concentration of generally about 0.1 to 100 mmol/l and preferably about 1 to 10 mmol/l. The phenylalanine or phenylpyruvic acid compounds are preferably incorporated within the concentration range of about 0.1 to 1% by weight.

In accordance with the method of this invention, the above-mentioned culture broth or living cells separated therefrom or dried cells obtainable therefrom are permitted to act on a lower alkyl ester of phenylpyruvic acid and an amino group donor selected from the group consisting of ammonium salts and ammonia in the presence of hydrogen, water and an organic solvent to give the desired L-phenylalanine lower alkyl ester. As the hydrogen partial pressure of the system is increased, the rate of production of the L-phenylalanine lower alkyl ester is generally increased. The hydrogen partial pressure is preferably not less than 0.5 atmosphere (absolute pressure), more preferably not less than 10 atmospheres (absolute) and still more preferably not less than 20 atmospheres (absolute). Furthermore, for the efficient production of L-phenylalanine lower alkyl esters on a commercial scale, the hydrogen partial pressure is preferably not less than 50 atmospheres (absolute) and more desirably not less than 80 atmospheres (absolute). There is virtually no upper limit to hydrogen partial pressure but in consideration of the cost of pressure-resistant reactor and other equipment required, the upper limit of hydrogen partial pressure is generally about 200 atmospheres (absolute) and preferably about 150 atmospheres (absolute).

The reaction system may contain certain gases other than hydrogen gas which would not interfere with the reaction according to the invention and as examples of such gases, there may be mentioned oxygen,

3

nitrogen, helium, argon, carbon dioxide and so on. There are cases in which the presence of oxygen in the reaction system contributes to a longer maintenance of the enzymatic activity of the cells. When oxygen is used in the reaction system, it is generally preferred that the oxygen partial pressure of the system is not more than 1 atmosphere (absolute pressure) and that the ratio of oxygen partial pressure to hydrogen partial pressure is not more than 0.3. For causing oxygen to be present in the reaction system, an oxygen precursor such as hydrogen peroxide may be added to the reaction system.

As examples of said lower alkyl esters of phenylpyruvic acid which are used in the practice of the invention, there may be mentioned various esters of phenylpyruvic acid with alkanols of 1 to 5 carbon atoms, such as methyl phenylpyruvate, ethyl phenylpyruvate, propyl phenylpyruvate, isopropyl phenylpyruvate, butyl phenylpyruvate, isobutyl phenylpyruvate, pentyl phenylpyruvate, and so on. Of these esters, methyl phenylpyruvate, ethyl phenylpyruvate, etc. are particularly desirable in view of the high production rate of the corresponding lower alkyl esters of L-phenyl alanine. The reaction for production of an L-phenylalanine lower alkyl ester in the presence of water and an organic solvent in accordance with the invention is carried out in a homogeneous liquid phase system comprising a uniform mixture of water and said organic solvent or a heterogeneous liquid phase system consisting of a water layer and an organic layer as will hereinafter be described in detail. The lower alkyl ester of phenylpyruvic acid is used in an amount assuring a concentration in said liquid phase in the range of generally about 1 to 200 mmol/l, preferably about 3 to 100 mmmol/l, and still more desirably about 5 to 80 mmol/l.

Examples of the amino group donor compound which is used in the practice of the invention include various ammonium salts such as ammonium acetate, ammonium chloride, ammonium nitrate, ammonium sulfate, ammonium carbonate, diammonium hydrogen phosphate, ammonium dihydrogen phosphate, etc. and ammonia. These amino group donors are used either singly or in a combination of two or more species. The appropriate amount of such amino group donor is generally in the range of about 1 to 100 moles per mole of the lower alkyl ester of phenylpyruvic acid used.

Representative examples of the organic solvent which is used in the reaction system in accordance with the invention include water-soluble or water-insoluble organic solvents, e.g. carboxylic acid esters such as ethyl acetate, methyl acetate, methyl propionate, etc.; alcohols such as methanol, ethanol, 1-propanol, 1-hexanol, ethylene glycol, diethylene glycol, etc.; ethers such as diethyl ether, ethyl propyl ether, anisole, ethylene glycol dimethyl ether, diethylene glycol dimethyl ether, tetrahydrofuran, dioxane, etc.; halogenated hydrocarbons such as chloroform, methylene chloride, etc.; nitriles such as acetonitrile, propionitrile, benzonitrile, etc.; various hydrocarbons including aliphatic hydrocarbons such as pentane, hexane, heptane, 2-pentene, 1-hexene, 1-octene, etc., alicyclic hydrocarbons such as cyclohexane, methylcyclohexane, cyclohexene, etc. and aromatic hydrocarbons such as benzene, toluene, xylene, etc.; ketones such as acetone, 2-butanone, 3-pentanone, 4-methyl-2-pentanone, 3-heptanone, etc.; sulfoxides such as dimethyl sulfoxide etc.; and so on. These solvents are used either singly or in a combination of two or more species. The amount of such organic solvent may generally range from about 0.1 to 100 times the volume of water present in the reaction system. For assuring a high rate of production of the desired L-phenylalanine lower alkyl ester, the amount of such organic solvent is preferably in the range of about 0.2 to 80 times the volume of water present in the reaction system and more desirably in the range of about 0.5 to 60 times the volume of water. In the reaction system, the organic solvent and water form either a homogeneous phase or heterogeneous phases but under either conditions an L-phenylalanine lower alkyl ester can be successfully produced.

This reaction is preferably conducted at a pH in the range of about 6 to 10 and still more desirably in the range of pH about 7 to 8.5. The pH of the reaction system may be adjusted by adding any of various buffer solutions such as Tris buffer, phosphate buffer, citrate buffer, tricine buffer, etc.; inorganic acids such as hydrochloric acid, sulfuric acid, phosphoric acid, etc.; acid, organic acids such as formic acid, acetic propionic acid, benzenesulfonic acid, etc.; hydroxides or carbonates of alkali metals or alkaline earth metals, such as sodium hydroxide, potassium hydroxide, barium hydroxide, sodium carbonate, potassium carbonate, sodium hydrogen carbonate, etc.; ammonia; and amines such as triethylamine and so on. In order that the ability of the microorganism to produce an L-phenylalanine lower alkyl ester may be augmented and maintained for an extended time period, it is preferable to incorporate in the reaction system various nutrient sources such as the carbon and nitrogen sources mentioned hereinbefore for cultivation of microorganisms belonging to the genus Nocardia. The reaction temperature may generally range from about 20 to 50°C and is preferably in the range of about 30 to 40°C. In certain cases, the presence of at least one co-enzyme selected from the group consisting of nicotinamide-adenine dinucleotide (hereinafter referred to as NAD) and reduced nicotinamide-adenine dinucleotide (hereinafter referred to as NADH) in the reaction system contributes to an increased production rate of L-phenylalanine lower alkyl esters. Such co-enzyme is used in an amount providing a concentration in said liquid phase of about 0.1 to 2.0 mmol/l in

4

the reaction system.

In accordance with the invention, the reaction for production of an L-phenylalanine lower alkyl ester can be conducted batchwise under stirring or shaking. The reaction can also be conducted continuously by feeding a mixture of said phenylpyruvic acid lower alkyl ester, amino group donor, water and organic solvent and a hydrogen gas, which may contain said other gases, to a columnar or vessel-type reactor containing said immobilized cells from time to time or continuously so as to allow the reaction to proceed under fluidization of the immobilized cells and withdrawing the reaction mixture containing the product L-phenylalanine lower alkyl ester from time to time or continuously or by passing a mixture of said phenylpyruvic acid lower alkyl ester, amino group donor, water and organic solvent and a hydrogen gas, which may contain said other gases, through a tubular reactor packed with the immobilized cells.

After completion of the reaction, the cells in the reaction mixture are separated by filtration or centrifugation. The L-phenylalanine lower alkyl ester in the filtrate or supernatant can then be isolated by ion exchange resin method, crystallization method and other procedures.

The immobilized cells recovered from the reaction mixture can be reused, if desired, for the production of L-phenylalanine lower alkyl esters in accordance with the invention. When the ability of the immobilized cells so recovered to produce L-phenylalanine lower alkyl esters is found to have been consumed and attenuated, it is possible to restore the same ability by treating the cells with an aqueous solution containing at least one of said metal salt, phenylalanine compound and phenylpyruvic acid compound in the same manner as described hereinbefore.

L-Phenylalanine lower alkyl esters are of value as starting materials for the synthesis of $\alpha$-L-aspartyl-L-phenylalanine lower alkyl esters which are represented by $\alpha$-L-aspartyl-L-phenylalanine methyl ester which is known as an artificial sweetener.

A further understanding of this invention can be obtained by reference to specific examples which are provided hereinbelow for purposes of illustration only and are not intended to be limitative of this invention.

Example 1

In purified water were dissolved 1.0 g of meat extract, 1.0 g of peptone, 0.5 g of sodium chloride and 0.4 g of L-phenylalanine methyl ester hydrochloride and the solution was adjusted to pH 7.2 with 1N-aqueous sodium hydroxide solution, followed by further addition of purified water to make 100 ml. A 500-ml Sakaguchi flask was charged with 100 m $\ell$ of the medium prepared above (which is referred to hereinafter as medium 1), which was then steam-sterilized at 120°C for 10 minutes. The strain Nocardia opaca C-8-5 was grown on an agar slant medium prepared by adding agar to a medium similar to the above medium 1 and a loopful of the strain was inoculated into medium 1 in said 500-m $\ell$ Sakaguchi flask and cultivated at 34°C for 10 hours. After cultivation, the cells were recovered by centrifugation and washed with 0.05 mol/l Tris-HCl buffer (pH 7.8).

The resulting cells of Nocardia opaca C-8-5 (1.5 g on a dry cell basis) were suspended in 40 ml of 0.05 mol/l Tris-HCl buffer (pH 7.8). This cell suspension was mixed with 40 m $\ell$ of an aqueous solution containing 4% (by weight) of sodium alginate and the mixture was dripped into an aqueous solution containing 0.5 mol/l of calcium chloride, whereby beads (diameter: about 3 mm) were obtained. A 1.5-cm$^3$ portion of the resulting beads containing immobilized cells of Nocardia opaca C-8-5 (30 mg of cells on a dry cell basis) were immersed in 10 ml of an aqueous solution prepared by adding 5 mmol/l of zinc chloride to medium 1 at 37°C for 36 hours, after which the beads were washed with 0.05 mol/l Tris-HCl buffer (pH 7.8).

Then, 8 m $\ell$ of ethyl acetate containing 12.5 mmol/l of methyl phenylpyruvate was mixed with 2 m $\ell$ of an aqueous solution prepared by adding ammonium chloride and NAD at the final concentrations of 0.5 mol/l and 4 mmol/l, respectively, and a stainless steel autoclave having a capacity of 50 m $\ell$ was charged with the resulting mixture and the above-prepared beads. After purging the internal atmosphere of the autoclave with hydrogen gas, the internal pressure was increased to 100 atmospheres (absolute pressure) with hydrogen gas. The reaction was then conducted at 37°C for 20 hours, with the hydrogen gas pressure being maintained at 100 atmospheres (absolute). After completion of the reaction, the ethyl acetate layer and the aqueous layer were analyzed by gas chromatography and high performance liquid chromatography, respectively. As a result, it was found that 9.8 mg of methyl phenylpyruvate remained unreacted and 6.4 mg of L-phenylalanine methyl ester had been produced. The rate of production of L-phenylalanine methyl ester was 1.0 $\mu$mol/min/g (dry cells). The yield of L-phenylalanine methyl ester based on the methyl phenylpyruvate used was 36% and the selectivity to L-phenylalanine methyl ester was 80%.

## Examples 2-12

The procedure of Example 1 was repeated except that 8 mℓ of one of the organic solvents mentioned in Table 1 and each containing 12.5 mmol/ℓ of methyl phenylpyruvate was used in lieu of 8 ml of ethyl acetate containing 12.5 mmol/ℓ of methyl phenylpyruvate to give L-phenylalanine methyl ester. Where the resulting reaction mixture was a binary-phase mixture consisting of an organic layer and an aqueous layer, the organic layer was analyzed by gas chromatography and the aqueous layer by high performance liquid chromatography and where the reaction mixture was a homogeneous system, it was analyzed by gas chromatography and high performance liquid chromatography to estimate the residual amount of methyl phenylpyruvate and the amount of L-phenylalanine methyl ester produced. The residual amount of methyl phenylpyruvate, the output of L-phenylalanine methyl ester, the rate of production of the ester, the yield of the product ester based on the methyl phenylpyruvate used, and the selectivity to L-phenylalanine methyl ester are shown in Table 1.

Table 1

| Example | Organic solvent | Methyl phenylpyruvate Residual amount (mg) | L-Phenylalanine methyl ester | | | |
|---|---|---|---|---|---|---|
| | | | Output (mg) | Production rate [$\mu$mol/min/g (dry cells)] | Yield (%) | Selectivity (%) |
| 2 | Methanol | 10.4 | 5.7 | 0.89 | 32 | 77 |
| 3 | Methyl acetate | 10.9 | 5.2 | 0.81 | 29 | 75 |
| 4 | Diethyl ether | 13.2 | 3.6 | 0.56 | 20 | 78 |
| 5 | Acetonitrile | 13.2 | 3.2 | 0.50 | 18 | 70 |
| 6 | Chloroform | 14.0 | 2.8 | 0.44 | 16 | 74 |
| 7 | Hexane | 14.5 | 2.6 | 0.40 | 15 | 79 |
| 8 | Ethanol | 13.2 | 3.5 | 0.55 | 20 | 76 |
| 9 | 3-Heptanone | 13.5 | 2.9 | 0.45 | 16 | 67 |
| 10 | Dimethyl sulfoxide | 14.4 | 2.2 | 0.34 | 12 | 65 |
| 11 | Cyclohexane | 14.6 | 2.4 | 0.37 | 13 | 75 |
| 12 | Ethyl acetatebenzene (4:1, v/v) | 13.4 | 3.0 | 0.47 | 17 | 68 |

## Example 13

The procedure of Example 1 was repeated except that 4 ml of ethyl acetate containing 25 mmol/l of methyl phenylpyruvate was used in lieu of 8 ml of ethyl acetate containing 12.5 mmol/l of methyl phenylpyruvate and that 6 ml of an aqueous solution prepared by adding ammonium chloride and NAD to medium 1 at the final concentrations of 0.17 mol/l and 1.3 mmol/l, respectively, was used in lieu of 2 ml of the aqueous solution prepared by adding ammonium chloride and NAD to medium 1 at the final concentrations of 0.5 mol/l and 4 mmol/l, respectively, whereby L-phenylalanine methyl ester was produced. The residual amount of methyl phenylpyruvate was 10.0 mg and the output of L-phenylalanine methyl ester was 6.0 mg. The rate of production of L-phenylalanine methyl ester was 0.93 $\mu$mol/min/g (dry cells). The yield of L-phenylalanine methyl ester based on the methyl phenylpyruvate used was 34% and the selectivity to L-phenylalanine methyl ester was 77%.

## Example 14

The procedure of Example 1 was repeated except that 10 ml of ethyl acetate containing 10 mmol/l of methyl phenylpyruvate was used in lieu of 8 ml of ethyl acetate containing 12.5 mmol/l of methyl phenylpyruvate and that 0.2 ml of an aqueous solution prepared by adding ammonium chloride and NAD to medium 1 at the final concentrations of 5.0 mol/l and 40 mmol/l, respectively, was used in lieu of 2 ml of the aqueous solution prepared by adding ammonium chloride and NAD to medium 1 at the final concentrations of 0.5 mol/l and 4 mmol/l, respectively, whereby L-phenylalanine methyl ester was produced. The residual amount of methyl phenylpyruvate was 11.2 mg and the output of L-phenylalanine methyl ester was 5.4 mg. The rate of production of L-phenylalanine methyl ester was 0.84 $\mu$mol/min/g (dry cells). The yield of L-phenylalanine methyl ester based on the methyl phenylpyruvate used was 30% and the selectivity to L-phenylalanine methyl ester was 82%.

## Example 15

The procedure of Example 1 was repeated except that 8 ml of ethyl acetate containing 12.5 mmol/l of ethyl phenylpyruvate was used in lieu of 8 ml of ethyl acetate containing 12.5 mmol/l of methyl phenylpyruvate to give L-phenylalanine ethyl ester. The residual amount of ethyl phenylpyruvate was 15.6 mg and the output of L-phenylalanine ethyl ester was 3.0 mg. The rate of production of L-phenylalanine ethyl ester was 0.43 $\mu$mol/min/g (dry cells). The yield of L-phenylalanine ethyl ester based on the ethyl phenylpyruvate used was 16% and the selectivity to L-phenylalanine ethyl ester was 83%.

## Example 16

The procedure of Example 1 was repeated except that 2 ml of an aqueous solution prepared by adding ammonium chloride and NAD to 0.1 mol/l Tris-HCl buffer (pH 8.0) at the final concentrations of 0.5 mol/l and 4 mmol/l, respectively, was used in lieu of 2 ml of the aqueous solution prepared by adding ammonium chloride and NAD to medium 1 at the final concentrations of 0.5 mol/l and 4 mmol/l, respectively, whereby L-phenylalanine methyl ester was produced. The residual amount of methyl phenylpyruvate was 10.7 mg and the output of L-phenylalanine methyl ester was 5.1 mg. The rate of production of L-phenylalanine methyl ester was 0.80 $\mu$mol/min/g (dry cells). The yield of L-phenylalanine methyl ester based on the methyl phenylpyruvate used was 29% and the selectivity to L-phenylalanine methyl ester was 72%.

## Examples 17 and 18

The procedure of Example 1 was repeated except that the hydrogen gas pressure was controlled at 50 or 2 atmospheres (absolute) instead of 100 atmospheres (absolute) to give L-phenylalanine methyl ester. The results are shown in Table 2.

Table 2

| Example | Hydrogen gas pressure [atmospheres (absolute pressure)] | Residual amount of methyl phenylpyruvate (mg) | L-Phenylalanine methyl ester | | | |
|---|---|---|---|---|---|---|
| | | | Output (mg) | Rate of production [μmol/min/g (dry cells)] | Yield (%) | Selectivity (%) |
| 17 | 50 | 13.2 | 3.4 | 0.53 | 19 | 74 |
| 18 | 2 | 16.1 | 0.9 | 0.14 | 5 | 53 |

EP 0 310 034 A2

Example 19

The cultivation and immobilization of microorganism and the reaction were carried out in the same manner as Example 1 except that the strain Nocardia erythropolis C-6-2 was used in lieu of the strain Nocardia opaca C-8-5. The residual amount of methyl phenylpyruvate was 10.5 mg and the output of L-phenylalanine methyl ester was 5.7 mg. The rate of production of L-phenylalanine methyl ester was 0.88 μmol/min/g (dry cells). The yield of L-phenylalanine methyl ester based on the methyl phenylpyruvate used was 32% and the selectivity to L-phenylalanine methyl ester was 78%.

Example 20

The cultivation and immobilization of microorganism and the reaction were carried out in the same manner as Example 1 except that the strain Nocardia coeliaca C-7-5 was used in lieu of the strain Nocardia opaca C-8-5. The residual amount of methyl phenylpyruvate was 12.1 mg and the output of L-phenyl alanine methyl ester was 4.7 mg. The rate of production of L-phenylalanine methyl ester was 0.73 μmol/min/g (dry cells). The yield of L-phenylalanine methyl ester based on the methyl phenylpyruvate used was 26% and the selectivity to L-phenylalanine methyl ester was 82%.

Examples 21-23

The procedure of Example 1 was repeated except that 2 ml of an aqueous solution obtained by adding one of the amino group donors mentioned in Table 3 and NAD to medium 1 at the concentration indicated in Table 3 and 4 mmol/l, respectively, in lieu of 2 ml of the aqueous solution prepared by adding ammonium chloride and NAD to medium 1 at the final concentrations of 0.5 mol/l and 4 mmol/l, respectively, whereby L-phenylalanine methyl ester was produced. The results are shown in Table 3.

Table 3

| Example | Amino group donor (mol/l) | Residual amount of methyl phenylpyruvate (mg) | L-Phenylalanine methyl ester | | | |
|---|---|---|---|---|---|---|
| | | | Output (mg) | Rate of production [$\mu$mol/min/g (dry cells)] | Yield (%) | Selectivity (%) |
| 21 | Ammonium acetate (0.5) | 10.2 | 6.1 | 0.95 | 34 | 80 |
| 22 | Ammonium sulfate (0.25) | 10.9 | 5.6 | 0.87 | 31 | 81 |
| 23 | Ammonia (0.5) | 10.9 | 5.5 | 0.86 | 31 | 80 |

## Example 24

The procedure of Example 1 was repeated except that 2 ml of an aqueous solution prepared by adding ammonium chloride and NADH to medium 1 at the final concentrations of 0.5 mol/l and 4 mmol/l, respectively, was used in lieu of 2 ml of the aqueous solution obtained by adding ammonium chloride and NAD to medium 1 at the final concentrations of 0.5 mol/l and 4 mmol/l, respectively, whereby L-phenylalanine methyl ester was produced. The residual amount of methyl phenylpyruvate was 9.0 mg and the output of L-phenylalanine methyl ester was 7.0 mg. The rate of production of L-phenylalanine methyl ester was 1.10 $\mu$mol/min/g (dry cells). The yield of L-phenylalanine methyl ester based on the methyl phenylpyruvate used was 39% and the selectivity to L-phenylalanine methyl ester was 80%.

## Example 25

The procedure of Example 1 was repeated except that 2 ml of an aqueous solution prepared by adding ammonium chloride to medium 1 at the final concentration of 0.5 mol/l was used in lieu of the aqueous solution obtained by adding ammonium chloride and NAD to medium 1 at the final concentrations of 0.5 mol/l and 4 mmol/l, respectively, whereby L-phenylalanine methyl ester was produced. The residual amount of methyl phenylpyruvate was 11.5 mg and the output of L-phenylalanine methyl ester was 5.0 mg. The rate of production of L-phenylalanine methyl ester was 0.78 $\mu$mol/min/g (dry cells). The yield of L-phenylalanine methyl ester based on the methyl phenylpyruvate used was 28% and the selectivity to L-phenylalanine methyl ester was 79%.

## Example 26

The procedure of Example 1 was repeated to give L-phenylalanine methyl ester (first reaction). Thereafter, the beads containing the cells were recovered and washed with 0.05 mol/l Tris-HCl buffer (pH 7.8). Then, the procedure of Example 1 was repeated except that the washed beads thus obtained were reused, whereby L-phenylalanine methyl ester was produced (second reaction). After this second reaction, the beads were recovered and washed in the same manner as above and the same reaction procedure was repeated further twice to produce L-phenylalanine methyl ester each time (third and fourth reactions). The results of the respective reactions are shown in Table 4.

Table 4

| Reaction No. | Residual amount of methyl phenylpyruvate (mg) | L-Phenylalanine methyl ester | | | |
|---|---|---|---|---|---|
| | | Output (mg) | Rate of production [$\mu$mol/min/g (dry cells)] | Yield (%) | Selectivity (%) |
| 1 | 9.8 | 6.4 | 1.00 | 36 | 80 |
| 2 | 11.8 | 4.8 | 0.75 | 27 | 80 |
| 3 | 12.4 | 4.2 | 0.65 | 24 | 78 |
| 4 | 13.9 | 3.1 | 0.48 | 17 | 79 |

Example 27

The procedure of Example 1 was repeated except that the cultured cells (30 mg on a dry cell basis) were used without immobilization in the reaction. The residual amount of methyl phenylpyruvate was 15.7 mg and the output of L-phenylalanine methyl ester was 1.6 mg. The rate of production of L-phenylalanine methyl ester was 0.25 $\mu$mol/min/g (dry cells). The yield of L-phenylalanine methyl ester based on the methyl phenylpyruvate used was 9% and the selectivity to L-phenylalanine methyl ester was 75%.

Comparative Example

The general procedure of Example 1 was repeated except that the reactor was charged with 18 mg (0.10 mmol) of methyl phenylpyruvate and 10 ml of an aqueous solution prepared by adding ammonium chloride and NAD to medium 1 at the final concentrations of 0.1 mol/l and 0.8 mmol/l, respectively, in lieu of 8 ml of ethyl acetate containing 12.5 mmol/l of methyl phenylpyruvate and 2 ml of an aqueous solution prepared by adding ammonium chloride and NAD to medium 1 at the final concentrations of 0.5 mol/l and 4 mmol/l, respectively, whereby L-phenylalanine methyl ester was produced. After completion of the reaction, 10 ml of ethyl acetate was added to the reaction mixture and the ethyl acetate layer and the aqueous layer were analyzed by gas chromatography and high performance liquid chromatography, respectively. The residual amount of methyl phenylpyruvate was 17.1 mg and the output of L-phenylalanine methyl ester was 0.5 mg. The rate of production of L-phenylalanine methyl ester was 0.08 $\mu$mol/min/g (dry cells). The yield of L-phenylalanine methyl ester based on the methyl phenylpyruvate used was 3% and the selectivity to L-phenylalanine methyl ester was 71%.

**Claims**

1. A process for producing an L-phenylalanine lower alkyl ester, which comprises permitting a microorganism which belongs to the genus Nocardia and is capable of producing an L-phenylalanine lower alkyl ester from the corresponding lower alkyl ester of phenylpyruvic acid and an amino group donor to act upon the lower alkyl ester of phenylpyruvic acid and an ammonium salt or ammonia as amino group donor in the presence of hydrogen, water and an organic solvent.

2. The process claimed in Claim 1 wherein the hydrogen partial pressure of the reaction system is not less than 0.5 atmosphere (absolute).

3. The process claimed in Claim 2 wherein the hydrogen partial pressure is not less than 10 atmospheres (absolute).

4. The process claimed in Claim 1 wherein said microorganism belongs to the species Nocardia opaca.

5. The process claimed in Claim 4 wherein said microorganism is the strain Nocardia opaca C-8-5 (FERM BP-1119).

6. The process claimed in Claim 1 wherein said microorganism belongs to the species Nocardia coeliaca.

7. The process claimed in Claim 6 wherein said microorganism is the strain Nocardia coeliaca C-7-5 (FERM BP-1117).

8. The process claimed in Claim 1 wherein said microorganism belongs to the species Nocardia erythropolis.

9. The process claimed in Claim 8 wherein said microorganism is the strain Nocardia erythropolis C-6-2 (FERM BP-1118).

10. The process claimed in Claim 1 wherein said microorganism is used as immobilized.

11. The process claimed in Claim 1 wherein said organic solvent is at least one carboxylic acid ester, alcohol, ether, halogenated hydrocarbon, nitrile, hydrocarbon, ketone and/or sulfoxide.

12. The process claimed in Claim 1 wherein at least one of the co-enzymes nicotinamide-adenine dinucleotide and reduced nicotinamide-adenine dinucleotide is added to the reaction system.